# EUROPEAN PATENT APPLICATION

(11) **EP 4 696 696 A1**
(43) Date of publication of application: **18.02.2026**
(21) Application number: 24788521.3
(22) Date of filing: 18.03.2024
(51) Int. Cl.: C07F 19/00, C07B 61/00, C07D 317/36, C07F 7/18, C07F 9/50

(54) **IMMOBILIZED QUATERNARY PHOSPHONIUM SALT HAVING PHENOL DOMAIN, METHOD FOR PRODUCING SAME, AND CYCLIC CARBONATE PRODUCTION METHOD USING SAME**

(30) Priority: 14.04.2023 JP 2023066124
(71) Applicant: SHIN-ETSU CHEMICAL CO., LTD., Tokyo 1000005 (JP)
(72) Inventor: ICHII, Shun, Joetsu-shi, Niigata 942-8601 (JP); TONOMURA, Yoichi, Joetsu-shi, Niigata 942-8601 (JP); KIYOMORI, Ayumu, Joetsu-shi, Niigata 942-8601 (JP)
(74) Representative: Mewburn Ellis LLP
(86) International application number: PCT/JP2024/010487
(87) International publication number: WO 2024/214491

(57) **Abstract**

This immobilized quaternary phosphonium salt having a phenol domain is formed of at least an inorganic carrier and a quaternary phosphonium silane that is represented by general formula (1) and that has a phenol domain. A silicon atom of the quaternary phosphonium silane having a phenol domain is immobilized to the inorganic carrier via a covalent bond with an oxygen atom existing on the surface of the inorganic carrier. The immobilized quaternary phosphonium salt having a phenol domain enables production of a cyclic carbonate compound at an excellent yield under a mild condition such as at ordinary pressure and/or room temperature, and can be easily recovered and recycled after reaction. 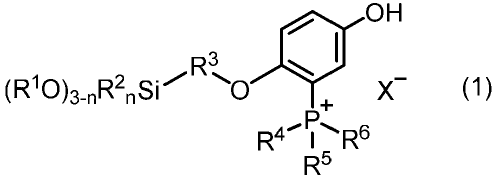 (R¹ and R² each represent a monovalent hydrocarbon group having 1-10 carbon atoms, R³ represents a divalent hydrocarbon group having 1-10 carbon atoms, R⁴-R⁶ each represent a monovalent hydrocarbon group having 1-10 carbon atoms, n represents an integer of 0-2, and X represents a halogen atom.)

## Description

### TECHNICAL FIELD

The present invention relates to an immobilized quaternary phosphonium salt having a phenol moiety, a method for preparing the same, and a method for synthesizing a cyclic carbonate using the same.

### BACKGROUND ART

Cyclic carbonate compounds such as ethylene carbonate and propylene carbonate are endowed with excellent properties, including high permittivity, ease of derivatization and low toxicity, making them useful as, for example, electrolyte solvents for lithium secondary batteries, starting materials for plastics such as polycarbonates and polyhydroxyurethanes, and resin plasticizers.

One method for synthesizing cyclic carbonate compounds is a reaction that effects the cycloaddition of an epoxide with carbon dioxide in the presence of a catalyst. Given the strong desire by society for carbon neutrality, this method, because it is able to fix carbon dioxide and convert it into useful compounds, is an important method for utilizing carbon dioxide.

Homogeneous catalysts such as quaternary ammonium salts and alkali metal salts have hitherto been used as the catalyst in the above cycloaddition reaction between an epoxide and carbon dioxide.

On the other hand, immobilized catalysts are useful because they have certain advantages lacking in homogeneous catalysts; for example, they can be easily separated from the reaction product such as by filtration, they are recoverable and reusable, and they can be employed in continuous synthesis. Specifically, by using an immobilized catalyst, it is possible to simplify the catalyst removal step, enhance productivity and atomic efficiency, and reduce waste products such as catalyst residues and solvents used in the reaction and in washing. Given such advantages, the application of immobilized catalysts to cycloaddition reactions between epoxides and carbon dioxide has been actively investigated in recent years.

Phosphonium bromide salt catalysts immobilized on silica gel are known as pioneering examples of immobilized catalysts. For instance, in Patent Document 1, cyclic carbonate compounds are produced from epoxides and carbon dioxide under 10 atmosphere, 90°C conditions using an alkyltriarylphosphonium bromide salt immobilized on silica gel as the catalyst. The catalyst in Patent Document 1 can be easily recovered by filtration following reaction completion, and can be reused without a marked decrease in catalytic activity. In Patent Document 2, using a tetraalkylphosphonium bromide salt immobilized on silica gel as the catalyst, propylene carbonate and ethylene carbonate are continuously synthesized by feeding epoxide and carbon dioxide under 70 atmosphere, 100°C conditions to a catalyst-packed reactor.

### PRIOR ART DOCUMENTS

### PATENT DOCUMENTS

Patent Document 1: JP-A 2008-296066
Patent Document 2: WO 2015/008854 A1

### SUMMARY OF INVENTION

### TECHNICAL PROBLEM

By using phosphonium bromide salt catalysts immobilized on silica gel, efforts have been made to achieve greater efficiency owing to the advantages of such immobilized catalysts, but these methods pose a challenge in that they require harsh reaction conditions. For example, in the case of the immobilized catalysts in Patent Documents 1 and 2, given their low catalytic activities, in order to synthesize a cyclic carbonate compound in a high yield, the reaction must be carried out at high pressure and high temperature, such as the 10 atmospheres and 90°C or the 70 atmospheres and 100°C mentioned above. In methods that require high-pressure and high-temperature reaction conditions such as this, not only is it necessary to use a large excess of carbon dioxide, enormous energy costs due to heating arise, which is a problem from the standpoint of sustainable production. Also, in Patent Document 2, because the catalyst is readily deactivated during the reaction, an alkyl bromide such as 2-bromoethanol must be separately added to suppress this. However, doing so is undesirable from the standpoint of atomic efficiency.

Accordingly, there exists a strong desire for the development of a highly active immobilized catalyst which, in the synthesis of a cyclic carbonate compound from an epoxide and carbon dioxide, is able to smoothly promote the reaction under mild reaction conditions such as normal pressure and/or room temperature and without the addition of additives.

The present invention was arrived at in light of the above circumstances. An object of the invention is to provide a phenol moiety-containing quaternary phosphonium salt immobilized on an inorganic support that can synthesize a cyclic carbonate compound in a good yield under mild conditions such as normal pressure and/or room temperature and moreover that can be easily recovered following the reaction and reused. Additional objects are to provide a method for preparing the immobilized quaternary phosphonium salt, and a method for synthesizing cyclic carbonate compounds using the immobilized quaternary phosphonium salt.

### SOLUTION TO PROBLEM

The inventors have conducted intensive investigations aimed at achieving the above objects. As a result, they have discovered that by using, in a cycloaddition reaction between an epoxide and carbon dioxide, an immobilized phenol moiety-containing quaternary phosphonium salt obtained by introducing a linker moiety having a phenol structure onto a quaternary phosphonium salt as a catalyst, the corresponding cyclic carbonate compound can be synthesized in a high yield and to a high purity under mild reaction conditions such as normal pressure (0.09 to 0.11 MPa) and/or room temperature (between 1°C and 30°C), and moreover that this immobilized zinc complex catalyst can be easily recovered and reused following reaction completion. This discovery ultimately led to the present invention.

Accordingly, the invention provides:
1. A phenol moiety-containing quaternary phosphonium salt immobilized on an inorganic support, which immobilized quaternary phosphonium salt comprises at least a phenol moiety-containing quaternary phosphonium silane of general formula (1) below 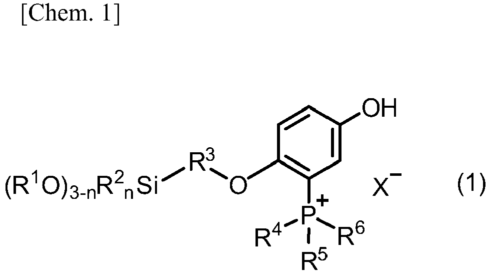 (wherein R¹ and R² are each independently a substituted or unsubstituted monovalent hydrocarbon group of 1 to 10 carbon atoms, R³ is an unsubstituted divalent hydrocarbon group of 1 to 10 carbon atoms that may include a heteroatom, R⁴ to R⁶ are each independently a substituted or unsubstituted monovalent hydrocarbon group of 1 to 10 carbon atoms, n is an integer from 0 to 2, and X is a halogen atom) and an inorganic support, wherein the quaternary phosphonium salt is immobilized on the inorganic support via covalent bonds between silicon atoms on the quaternary phosphonium silane and oxygen atoms present on a surface of the inorganic support;
2. A method for preparing an immobilized, phenol moiety-containing quaternary phosphonium salt of 1, which method includes the steps of, in order:
   reacting a haloalkyl alkoxysilane compound of general formula (2) below 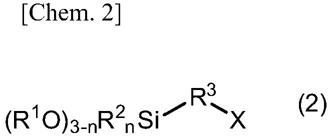
      (wherein R¹ to R³, X and n are as defined above)
      with a phosphabetaine compound of general formula (3) below 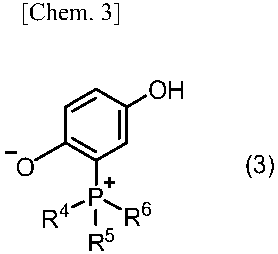
      (wherein R⁴ to R⁶ are as defined above)
      to form a phenol moiety-containing quaternary phosphonium silane of general formula (1) below 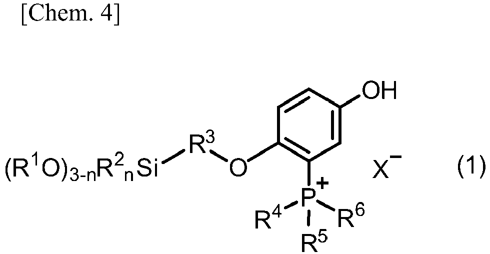
      (wherein R¹ to R⁶, X and n are as defined above); and
   mixing the resulting phenol moiety-containing quaternary phosphonium silane with an inorganic support to form covalent bonds between silicon atoms on the phenolic moiety-containing quaternary phosphonium silane and oxygen atoms present on a surface of the inorganic support, thereby immobilizing the quaternary phosphonium silane on the inorganic support; and
3. A method for synthesizing a cyclic carbonate compound of general formula (7) below 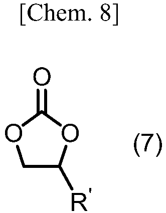
   [wherein R' is a substituted or unsubstituted monovalent hydrocarbon group of 1 to 18 carbon atoms which may include a heteroatom, a group of general formula (5) below
      [Chem. 9]

      -R⁷-SiR⁸m(OR⁹)₃₋ₘ (5)
   (wherein R⁷ is an unsubstituted divalent hydrocarbon group of 1 to 10 carbon atoms which may include a heteroatom, R⁸ and R⁹ are each independently a substituted or unsubstituted monovalent hydrocarbon group of 1 to 10 carbon atoms, and m is an integer from 0 to 3) or a group of general formula (8) below 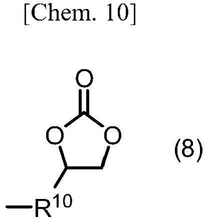
   (wherein R¹⁰ is a substituted or unsubstituted divalent hydrocarbon group of 1 to 20 carbon atoms which may include a heteroatom)], which method includes the step of carrying out, in the presence of a catalyst, a cycloaddition reaction between an epoxide of general formula (4) below 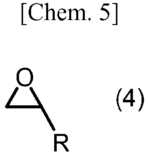
   [wherein R is a substituted or unsubstituted monovalent hydrocarbon group of 1 to 18 carbon atoms which may include a heteroatom, a group of general formula (5) below
      [Chem. 6]

      -R⁷-SiR⁸ₘ(OR⁹)₃₋ₘ (5)
   (wherein R⁷ to R⁹ and m are as defined above) or a group of general formula (6) below 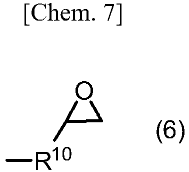
   (wherein R¹⁰ is as defined above)] and carbon dioxide,
      wherein the catalyst used to synthesize the cyclic carbonate compound is the immobilized, phenol moiety-containing quaternary phosphonium salt of 1 above.

### ADVANTAGEOUS EFFECTS OF INVENTION

This invention enables an immobilized, phenol moiety-containing quaternary phosphonium salt to be obtained. By using this immobilized, phenol moiety-containing quaternary phosphonium salt as a catalyst, cyclic carbonate compounds can be synthesized in a high yield and to a high purity under mild conditions such as normal pressure and/or room temperature. In addition, the phenol moiety-containing quaternary phosphonium salt immobilized on an inorganic support that is used as the catalyst can be easily recovered following reaction completion and reused.

### BRIEF DESCRIPTION OF DRAWINGS

[FIG. 1] FIG. 1 is an IR spectrum of the phenol moiety-containing quaternary phosphonium salt obtained in Example 1.
[FIG. 2] FIG. 2 is an IR spectrum of (2-[3-(trimethoxysilyl)propoxy]-5-hydroxyphenyl)triphenylphosphonium bromide prior to immobilization.
[FIG. 3] FIG. 3 is an ¹H-NMR spectrum of the 4,4'-[(2,2-dimethyl-1,3-propanediyl)bis(oxymethylene)]bis(1,3-dioxolan-2-one) obtained in Example 2-6.

### DESCRIPTION OF EMBODIMENTS

The invention is described in detail below.

The immobilized phenol moiety-containing quaternary phosphonium salt of the invention is comprised of at least a phenol moiety-containing quaternary phosphonium silane compound of general formula (1) below (referred to below as "Compound (1)") and an inorganic support, the phenolic moiety-containing quaternary phosphonium salt being immobilized on the inorganic support via covalent bonds between silicon atoms on Compound (1) and oxygen atoms present on the surface of the inorganic support.

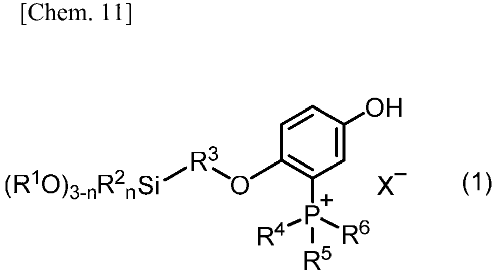

In general formula (1), R¹ and R² are each independently a substituted or unsubstituted monovalent hydrocarbon group of 1 to 10 carbon atoms, preferably 1 to 5 carbon atoms, and more preferably 1 to 3 carbon atoms.

The monovalent hydrocarbon groups represented by R¹ and R² may be linear, branched or cyclic. Specific examples include linear alkyl groups such as methyl, ethyl, n-propyl, n-butyl, n-pentyl, n-hexyl, n-heptyl, n-octyl, n-nonyl and n-decyl groups; branched alkyl groups such as isopropyl, isobutyl, sec-butyl, tert-butyl, isopentyl, neopentyl, isohexyl, isoheptyl, isooctyl and tert-octyl groups; cyclic alkyl groups such as cyclopentyl and cyclohexyl groups; alkenyl groups such as vinyl, allyl, 1-propenyl, butenyl and methallyl (2-methyl-2-propenyl) groups; aryl groups such as phenyl, tolyl and xylyl groups; and aralkyl groups such as benzyl and phenethyl groups.

Of these, R¹ and R² are preferably substituted or unsubstituted linear, branched or cyclic alkyl groups of 1 to 5 carbon atoms, alkenyl groups, aryl groups or aralkyl groups. Particularly from the standpoint of the availability of the starting materials, unsubstituted linear alkyl groups of 1 to 3 carbon atoms are more preferred; methyl and ethyl groups are even more preferred.

Some or all of the hydrogen atoms on these monovalent hydrocarbon groups may be replaced with other substituents, the substituents being exemplified by alkoxy groups of 1 to 3 carbon atoms such as methoxy, ethoxy and propoxy groups; halogen atoms such as fluorine, chlorine and bromine; aryl groups of 6 to 10 carbon atoms such as phenyl and tolyl groups; aralkyl groups of 7 to 10 carbon atoms such as benzyl and phenethyl groups; and also cyano groups, amino groups, ester groups, ether groups, carbonyl groups, acyl groups and sulfide groups. One of these may be used alone or two or more may be used in combination. The substitution sites for these substituents are not particularly limited. Nor is there any limit on the number of substituents.

In above general formula (1), R³ is an unsubstituted divalent hydrocarbon group of 1 to 10 carbon atoms, preferably 1 to 8 carbon atoms, more preferably 1 to 5 carbon atoms, and even more preferably 1 to 3 carbon atoms, which may include a heteroatom.

The divalent hydrocarbon group of R³ may be linear, branched or cyclic. Specific examples include alkylene groups such as methylene, ethylene, trimethylene, tetramethylene, isobutylene, hexamethylene, octamethylene, decamethylene, cyclohexylene and methylenecyclohexylene groups; alkenylene groups such as butynylene, propenylene, butenylene, hexenylene and octenylene groups; arylene groups such as the phenylene group; and aralkylene groups such as methylenephenylene and methylenephenylenemethylene groups.

Of these, R³ is preferably an unsubstituted linear alkylene group of 1 to 8 carbon atoms. Particularly from the standpoint of the availability of the starting materials, methylene, trimethylene and octamethylene groups are more preferred.

These divalent hydrocarbon groups may have one, two or more types of heteroatom interposed on the molecular chain, as in the case of ether, carbonyl, amino and sulfide groups.

In above general formula (1), R⁴ to R⁶ are each independently a substituted or unsubstituted monovalent hydrocarbon group of 1 to 10 carbon atoms, preferably 1 to 8 carbon atoms, and more preferably 1 to 6 carbon atoms.

The monovalent hydrocarbon groups of R⁴ to R⁶ may be linear, branched or cyclic. Specific examples include linear alkyl groups such as methyl, ethyl, n-propyl, n-butyl, n-pentyl, n-hexyl, n-heptyl, n-octyl, n-nonyl and n-decyl groups; branched alkyl groups such as isopropyl, isobutyl, sec-butyl, tert-butyl, decyl (1,1,2-trimethylpropyl) and 2-ethylhexyl groups; cyclic alkyl groups such as cyclopentyl and cyclohexyl groups; alkenyl groups such as vinyl, allyl and 1-propenyl groups; aryl groups such as phenyl and tolyl groups; and aralkyl groups such as the benzyl group. Of these, from the standpoint of the availability of the starting materials, methyl, n-butyl, tert-butyl, cyclohexyl, phenyl and tolyl groups are preferred.

In above general formula (1), X is a halogen atom such as fluorine, chlorine, bromine or iodine. From the standpoint of the availability of the starting materials, chlorine, bromine and iodine are preferred. For both catalytic activity and stability, bromine is especially preferred.

Specific examples of Compound (1) include (2-[(trialkoxysilyl)alkoxy]-5-hydroxyphenyl)triarylphosphonium bromides such as (2-[(trimethoxysilyl)methoxy]-5-hydroxyphenyl)triphenylphosphonium bromide, (2-[3-(trimethoxysilyl)propoxy]-5-hydroxyphenyl)triphenylphosphonium bromide, (2-[8-(trimethoxysilyl)octoxy]-5-hydroxyphenyl)triphenylphosphonium bromide, (2-[(trimethoxysilyl)methoxy]-5-hydroxyphenyl)tri(p-tolyl)phosphonium bromide, (2-[3-(trimethoxysilyl)propoxy]-5-hydroxyphenyl)tri(p-tolyl)phosphonium bromide, (2-[8-(trimethoxysilyl)octoxy]-5-hydroxyphenyl)tri(p-tolyl)phosphonium bromide, (2-[(triethoxysilyl)methoxy]-5-hydroxyphenyl)triphenylphosphonium bromide, (2-[3-(triethoxysilyl)propoxy]-5-hydroxyphenyl)triphenylphosphonium bromide, (2-[8-(triethoxysilyl)octoxy]-5-hydroxyphenyl)triphenylphosphonium bromide, (2-[(triethoxysilyl)methoxy]-5-hydroxyphenyl)tri(p-tolyl)phosphonium bromide, (2-[3-(triethoxysilyl)propoxy]-5-hydroxyphenyl)tri(p-tolyl)phosphonium bromide and (2-[8-(triethoxysilyl)octoxy]-5-hydroxyphenyl)tri(p-tolyl)phosphonium bromide; (2-[(dialkoxyalkylsilyl)alkoxy]-5-hydroxyphenyl)triarylphosphonium bromides such as (2-[(dimethoxymethylsilyl)methoxy]-5-hydroxyphenyl)triphenylphosphonium bromide, (2-[3-(dimethoxymethylsilyl)propoxy]-5-hydroxyphenyl)triphenylphosphonium bromide, (2-[8-(dimethoxymethylsilyl)octoxy]-5-hydroxyphenyl)triphenylphosphonium bromide, (2-[(dimethoxymethylsilyl)methoxy]-5-hydroxyphenyl)tri(p-tolyl)phosphonium bromide, (2-[3-(dimethoxymethylsilyl)propoxy]-5-hydroxyphenyl)tri(p-tolyl)phosphonium bromide, (2-[8-(dimethoxymethylsilyl)octoxy]-5-hydroxyphenyl)tri(p-tolyl)phosphonium bromide, (2-[(diethoxymethylsilyl)methoxy]-5-hydroxyphenyl)triphenylphosphonium bromide, (2-[3-(diethoxymethylsilyl)propoxy]-5-hydroxyphenyl)triphenylphosphonium bromide, (2-[8-(diethoxymethylsilyl)octoxy]-5-hydroxyphenyl)triphenylphosphonium bromide, (2-[(diethoxymethylsilyl)methoxy]-5-hydroxyphenyl)tri(p-tolyl)phosphonium bromide, (2-[3-(diethoxymethylsilyl)propoxy]-5-hydroxyphenyl)tri(p-tolyl)phosphonium bromide and (2-[8-(diethoxymethylsilyl)octoxy]-5-hydroxyphenyl)tri(p-tolyl)phosphonium bromide; (2-[(alkoxydialkylsilyl)alkoxy]-5-hydroxyphenyl)triarylphosphonium bromides such as (2-[(methoxydimethylsilyl)methoxy]-5-hydroxyphenyl)triphenylphosphonium bromide, (2-[3-(methoxydimethylsilyl)propoxy]-5-hydroxyphenyl)triphenylphosphonium bromide, (2-[8-(methoxydimethylsilyl)octoxy]-5-hydroxyphenyl)triphenylphosphonium bromide, (2-[(methoxydimethylsilyl)methoxy]-5-hydroxyphenyl)tri(p-tolyl)phosphonium bromide, (2-[3-(methoxydimethylsilyl)propoxy]-5-hydroxyphenyl)tri(p-tolyl)phosphonium bromide, (2-[8-(methoxydimethylsilyl)octoxy]-5-hydroxyphenyl)tri(p-tolyl)phosphonium bromide, (2-[(ethoxydimethylsilyl)methoxy]-5-hydroxyphenyl)triphenylphosphonium bromide, (2-[3-(ethoxydimethylsilyl)propoxy]-5-hydroxyphenyl)triphenylphosphonium bromide, (2-[8-(ethoxydimethylsilyl)octoxy]-5-hydroxyphenyl)triphenylphosphonium bromide, (2-[(ethoxydimethylsilyl)methoxy]-5-hydroxyphenyl)tri(p-tolyl)phosphonium bromide, (2-(3-ethoxydimethylsilyl)propoxy)-5-hydroxyphenyl)tri(p-tolyl)phosphonium bromide and (2-[8-(ethoxydimethylsilyl)octoxy]-5-hydroxyphenyl)tri(p-tolyl)phosphonium bromide; (2-[(trialkoxysilyl)alkoxy]-5-hydroxyphenyl)trialkylphosphonium bromides such as (2-[(trimethoxysilyl)methoxy]-5-hydroxyphenyl)trimethylphosphonium bromide, (2-[3-(trimethoxysilyl)propoxy]-5-hydroxyphenyl)trimethylphosphonium bromide, (2-[8-(trimethoxysilyl)octoxy]-5-hydroxyphenyl)trimethylphosphonium bromide, (2-[(trimethoxysilyl)methoxy]-5-hydroxyphenyl)tri(n-butyl)phosphonium bromide, (2-[3-(trimethoxysilyl)propoxy]-5-hydroxyphenyl)tri(n-butyl)phosphonium bromide, (2-[8-(trimethoxysilyl)octoxy]-5-hydroxyphenyl)tri(n-butyl)phosphonium bromide, (2-[(trimethoxysilyl)methoxy]-5-hydroxyphenyl)tri(tert-butyl)phosphonium bromide, (2-[3-(trimethoxysilyl)propoxy]-5-hydroxyphenyl)tri(tert-butyl)phosphonium bromide, (2-[8-(trimethoxysilyl)octoxy]-5-hydroxyphenyl)tri(tert-butyl)phosphonium bromide, (2-[(trimethoxysilyl)methoxy]-5-hydroxyphenyl)tri(cyclohexyl)phosphonium bromide, (2-[3-(trimethoxysilyl)propoxy]-5-hydroxyphenyl)tri(cyclohexyl)phosphonium bromide, (2-[8-(trimethoxysilyl)octoxy]-5-hydroxyphenyl)tri(cyclohexyl)phosphonium bromide, (2-[(triethoxysilyl)methoxy]-5-hydroxyphenyl)trimethylphosphonium bromide, (2-[3-(triethoxysilyl)propoxy]-5-hydroxyphenyl)trimethylphosphonium bromide, (2-[8-(triethoxysilyl)octoxy]-5-hydroxyphenyl)trimethylphosphonium bromide, (2-[(triethoxysilyl)methoxy]-5-hydroxyphenyl)tri(n-butyl)phosphonium bromide, (2-[3-(triethoxysilyl)propoxy]-5-hydroxyphenyl)tri(n-butyl)phosphonium bromide, (2-[8-(triethoxysilyl)octoxy]-5-hydroxyphenyl)tri(n-butyl)phosphonium bromide, (2-[(triethoxysilyl)methoxy]-5-hydroxyphenyl)tri(tert-butyl)phosphonium bromide, (2-[3-(triethoxysilyl)propoxy]-5-hydroxyphenyl)tri(tert-butyl)phosphonium bromide, (2-[8-(triethoxysilyl)octoxy]-5-hydroxyphenyl)tri(tert-butyl)phosphonium bromide, (2-[(triethoxysilyl)methoxy]-5-hydroxyphenyl)tricyclohexylphosphonium bromide, (2-[3-triethoxysilyl]propoxy)-5-hydroxyphenyl)tricyclohexylphosphonium bromide and (2-[8-(triethoxysilyl)octoxy]-5-hydroxyphenyl)tricyclohexylphosphonium bromide; (2-[(dialkoxyalkylsilyl)alkoxy]-5-hydroxyphenyl)trialkylphosphonium bromides such as (2-[(dimethoxymethylsilyl)methoxy]-5-hydroxyphenyl)trimethylphosphonium bromide, (2-[3-(dimethoxymethylsilyl)propoxy]-5-hydroxyphenyl)trimethylphosphonium bromide, (2-[8-(dimethoxymethylsilyl)octoxy]-5-hydroxyphenyl)trimethylphosphonium bromide, (2-[(dimethoxymethylsilyl)methoxy]-5-hydroxyphenyl)tri(n-butyl)phosphonium bromide, (2-[3-(dimethoxymethylsilyl)propoxy]-5-hydroxyphenyl)tri(n-butyl)phosphonium bromide, (2-[8-(dimethoxymethylsilyl)octoxy]-5-hydroxyphenyl)tri(n-butyl)phosphonium bromide, (2-[(dimethoxymethylsilyl)methoxy]-5-hydroxyphenyl)tri(tert-butyl)phosphonium bromide, (2-[3-(dimethoxymethylsilyl)propoxy]-5-hydroxyphenyl)tri(tert-butyl)phosphonium bromide, (2-[8-(dimethoxymethylsilyl)octoxy]-5-hydroxyphenyl)tri(tert-butyl)phosphonium bromide, (2-[(dimethoxymethylsilyl)methoxy]-5-hydroxyphenyl)tri(cyclohexyl)phosphonium bromide, (2-[3-(dimethoxymethylsilyl)propoxy]-5-hydroxyphenyl)tri(cyclohexyl)phosphonium bromide, (2-[8-(dimethoxymethylsilyl)octoxy]-5-hydroxyphenyl)tri(cyclohexyl)phosphonium bromide, (2-[(diethoxymethylsilyl)methoxy]-5-hydroxyphenyl)trimethylphosphonium bromide, (2-[3-(diethoxymethylsilyl)propoxy]-5-hydroxyphenyl)trimethylphosphonium bromide, (2-[8-(diethoxymethylsilyl)octoxy]-5-hydroxyphenyl)trimethylphosphonium bromide, (2-[(diethoxymethylsilyl)methoxy]-5-hydroxyphenyl)tri(n-butyl)phosphonium bromide, (2-[3-(diethoxymethylsilyl)propoxy]-5-hydroxyphenyl)tri(n-butyl)phosphonium bromide, (2-[8-(diethoxymethylsilyl)octoxy]-5-hydroxyphenyl)tri(n-butyl)phosphonium bromide, (2-[(diethoxymethylsilyl)methoxy]-5-hydroxyphenyl)tri(tert-butyl)phosphonium bromide, (2-[3-(diethoxymethylsilyl)propoxy]-5-hydroxyphenyl)tri(tert-butyl)phosphonium bromide, (2-[8-(diethoxymethylsilyl)octoxy]-5-hydroxyphenyl)tri(tert-butyl)phosphonium bromide, (2-[(diethoxymethylsilyl)methoxy]-5-hydroxyphenyl)tricyclohexylphosphonium bromide, (2-[3-(diethoxymethylsilyl)propoxy]-5-hydroxyphenyl)tricyclohexylphosphonium bromide and (2-[8-(diethoxymethylsilyl)octoxy]-5-hydroxyphenyl)tricyclohexylphosphonium bromide; and (2-[(alkoxydialkylsilyl)alkoxy]-5-hydroxyphenyl)trialkylphosphonium bromides such as (2-[(methoxydimethylsilyl)methoxy]-5-hydroxyphenyl)trimethylphosphonium bromide, (2-[3-(methoxydimethylsilyl)propoxy]-5-hydroxyphenyl)trimethylphosphonium bromide, (2-[8-(methoxydimethylsilyl)octoxy]-5-hydroxyphenyl)trimethylphosphonium bromide, (2-[(methoxydimethylsilyl)methoxy]-5-hydroxyphenyl)tri(n-butyl)phosphonium bromide, (2-[3-(methoxydimethylsilyl)propoxy]-5-hydroxyphenyl)tri(n-butyl)phosphonium bromide, (2-[8-(methoxydimethylsilyl)octoxy]-5-hydroxyphenyl)tri(n-butyl)phosphonium bromide, (2-[(methoxydimethylsilyl)methoxy]-5-hydroxyphenyl)tri(tert-butyl)phosphonium bromide, (2-[3-(methoxydimethylsilyl)propoxy]-5-hydroxyphenyl)tri(tert-butyl)phosphonium bromide, (2-[8-(methoxydimethylsilyl)octoxy]-5-hydroxyphenyl)tri(tert-butyl)phosphonium bromide, (2-[(methoxydimethylsilyl)methoxy]-5-hydroxyphenyl)tri(cyclohexyl)phosphonium bromide, (2-[3-(methoxydimethylsilyl)propoxy]-5-hydroxyphenyl)tri(cyclohexyl)phosphonium bromide, (2-[8-(methoxydimethylsilyl)octoxy]-5-hydroxyphenyl)tri(cyclohexyl)phosphonium bromide, (2-[(ethoxydimethylsilyl)methoxy]-5-hydroxyphenyl)trimethylphosphonium bromide, (2-[3-(ethoxydimethylsilyl)propoxy]-5-hydroxyphenyl)trimethylphosphonium bromide, (2-[8-(ethoxydimethylsilyl)octoxy]-5-hydroxyphenyl)trimethylphosphonium bromide, (2-[(ethoxydimethylsilyl)methoxy]-5-hydroxyphenyl)tri(n-butyl)phosphonium bromide, (2-[3-(ethoxydimethylsilyl)propoxy]-5-hydroxyphenyl)tri(n-butyl)phosphonium bromide, (2-[8-(ethoxydimethylsilyl)octoxy]-5-hydroxyphenyl)tri(n-butyl)phosphonium bromide, (2-[(ethoxydimethylsilyl)methoxy]-5-hydroxyphenyl)tri(tert-butyl)phosphonium bromide, (2-[3-(ethoxydimethylsilyl)propoxy]-5-hydroxyphenyl)tri(tert-butyl)phosphonium bromide, (2-[8-(ethoxydimethylsilyl)octoxy]-5-hydroxyphenyl)tri(tert-butyl)phosphonium bromide, (2-[(ethoxydimethylsilyl)methoxy]-5-hydroxyphenyl)tricyclohexylphosphonium bromide, (2-[3-(ethoxydimethylsilyl)propoxy]-5-hydroxyphenyl)tricyclohexylphosphonium bromide and (2-[8-(ethoxydimethylsilyl)octoxy]-5-hydroxyphenyl)tricyclohexylphosphonium bromide.

Of these, from the standpoint of the availability of the starting materials, (2-[3-(trialkoxysilyl)propoxy]-5-hydroxyphenyl)triphenylphosphonium bromides, (2-[3-(dialkoxyalkylsilyl)propoxy]-5-hydroxyphenyl)triphenylphosphonium bromides and (2-[3-(alkoxydialkylsilyl)propoxy]-5-hydroxyphenyl)triphenylphosphonium bromides are preferred; (2-[3-(trimethoxysilyl)propoxy]-5-hydroxyphenyl)triphenylphosphonium bromide, (2-[3-(triethoxysilyl)propoxy]-5-hydroxyphenyl)triphenylphosphonium bromide, (2-[3-(dimethoxymethylsilyl)propoxy]-5-hydroxyphenyl)triphenylphosphonium bromide, (2-[3-(diethoxymethylsilyl)propoxy]-5-hydroxyphenyl)triphenylphosphonium bromide, (2-[3-(methoxydimethylsilyl)propoxy]-5-hydroxyphenyl)triphenylphosphonium bromide and (2-[3-(ethoxydimethylsilyl)propoxy]-5-hydroxyphenyl)triphenylphosphonium bromide are more preferred.

The inorganic support used in this invention may be any material having hydroxyl groups on the surface, such as silica gel, mesoporous silica, alumina, zeolite, titania, ceria, zirconia and magnetite. From the standpoint of the availability of the starting materials, silica gel, mesoporous silica, alumina and zeolite are preferred.

Next, the method for preparing a quaternary phosphonium silane having a phenol moiety is described.

The immobilized phenol moiety-containing quaternary phosphonium salt of the invention can be obtained by reacting a haloalkylalkoxysilane compound of general formula (2) below (referred to below as "Compound (2)") with a phosphabetaine compound of general formula (3) below (referred to below as "Compound (3)") in the presence of an optional organic solvent so as to form a phenol moiety-containing quaternary phosphonium silane (Compound (1)), and then mixing the resulting phenol moiety-containing quaternary phosphonium silane with an inorganic support, thereby inducing the formation of covalent bonds between silicon atoms on the phenol moiety-containing quaternary phosphonium silane and oxygen atoms on the surface of the inorganic support and thus immobilizing the phenol moiety-containing quaternary phosphonium salt on the inorganic support. 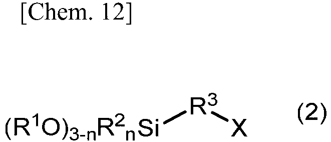 Here, R¹ to R³, X and n are as defined above. 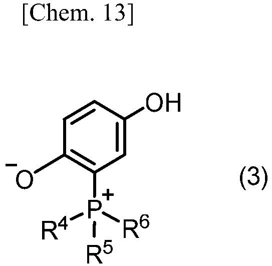 Here, R⁴ to R⁶ are as defined above.

Specific examples of Compound (2) include (bromoalkyl)trialkoxysilanes such as (bromomethyl)trimethoxysilane, (3-bromopropyl)trimethoxysilane, (8-bromooctyl)trimethoxysilane, (bromomethyl)triethoxysilane, (3-bromopropyl)triethoxysilane and (8-bromooctyl)triethoxysilane; (bromoalkyl)dialkoxyalkylsilanes such as (bromomethyl)dimethoxymethylsilane, (3-bromopropyl)dimethoxymethylsilane, (8-bromooctyl)dimethoxymethylsilane, (bromomethyl)diethoxymethylsilane, (3-bromopropyl)diethoxymethylsilane and (8-bromooctyl)diethoxymethylsilane; and (bromoalkyl)alkoxydialkylsilanes such as (bromomethyl)methoxydimethylsilane, (3-bromopropyl)methoxydimethylsilane, (8-bromooctyl)methoxydimethylsilane, (bromomethyl)ethoxydimethylsilane, (3-bromopropyl)ethoxydimethylsilane and (8-bromooctyl)ethoxydimethylsilane.

Of these, from the standpoint of the availability of the starting materials, (3-bromopropyl)alkoxysilanes are preferred; (3-bromopropyl)trimethoxysilane, (3-bromopropyl)triethoxysilane, (3-bromopropyl)dimethoxymethylsilane, (3-bromopropyl)diethoxymethylsilane, (3-bromopropyl)methoxydimethylsilane and (3-bromopropyl)ethoxydimethylsilane are more preferred.

A commercially available product may be used as Compound (2), or Compound (2) may be synthesized. In cases where it is synthesized, Compound (2) can be obtained in accordance with a known method, such as that of subjecting a halogenated alkenyl compound and a hydrosilane compound to a hydrosilylation reaction.

The amount of Compound (2) used in the above reaction is not particularly limited, although it is preferably from 0.9 to 1.1 mole, and more preferably from 0.95 to 1.05 mole, per mole of Compound (3).

Specific examples of Compound (3) include 4-hydroxy-2-(trialkylphosphonium) phenolates such as 4-hydroxy-2-(trimethylphosphonium) phenolate, 4-hydroxy-2-(triethylphosphonium) phenolate, 4-hydroxy-2-[tri(n-propyl)phosphonium] phenolate, 4-hydroxy-2-[tri(iso-propyl)phosphonium] phenolate, 4-hydroxy-2-[tri(n-butyl)phosphonium] phenolate, 4-hydroxy-2-[tri(tert-butyl)phosphonium] phenolate, 4-hydroxy-2-(tricyclopentylphosphonium) phenolate, 4-hydroxy-2-(trihexylphosphonium) phenolate, 4-hydroxy-2-(tricyclohexylphosphonium) phenolate and 4-hydroxy-2-(trioctylphosphonium) phenolate; 4-hydroxy-2-(triarylphosphonium) phenolates such as 4-hydroxy-2-(triphenylphosphonium) phenolate, 4-hydroxy-2-[tri(p-tolyl)phosphonium] phenolate, 4-hydroxy-2-[tri(m-tolyl)phosphonium] phenolate, 4-hydroxy-2-[tri(o-tolyl)phosphonium] phenolate, 4-hydroxy-2-[tri(p-methoxyphenyl)phosphonium] phenolate, 4-hydroxy-2-[tri(o-methoxyphenyl)phosphonium] phenolate and 4-hydroxy-2-[tri(p-fluorophenyl)phosphonium] phenolate; 4-hydroxy-2-[alkyldiarylphosphonium] phenolates such as 4-hydroxy-2-(methyldiphenylphosphonium) phenolate, 4-hydroxy-2-(ethyldiphenylphosphonium) phenolate, 4-hydroxy-2-[(n-propyl)diphenylphosphonium] phenolate, 4-hydroxy-2-[(iso-propyl)diphenylphosphonium] phenolate and 4-hydroxy-2-(cyclohexyldiphenylphosphonium) phenolate; and 4-hydroxy-2-(dialkylarylphosphonium) phenolates such as 4-hydroxy-2-(diethylphenylphosphonium) phenolate, 4-hydroxy-2-[di(tert-butyl)phenylphosphonium] phenolate and 4-hydroxy-2-(dicyclohexylphenylphosphonium) phenolate.

Of these, from the standpoint of the availability of the starting materials, 4-hydroxy-2-(triarylphosphonium) phenolates are preferred, and 4-hydroxy-2-(triphenylphosphonium) phenolate is more preferred.

A commercially available product may be used as Compound (3), or Compound (3) may be synthesized. In cases where it is synthesized, Compound (3) can be obtained in accordance with a known method, such as that of subjecting a tri-substituted phosphine compound and 1,4-benzoquinone to an addition reaction.

Examples of the organic solvent that may be optionally used in the above Compound (1) forming step include hydrocarbon solvents such as benzene, toluene and xylene; ether solvents such as diethyl ether, tetrahydrofuran, 2-methyltetrahydrofuran, 4-methyltetrahydropyran and dioxane; ester solvents such as ethyl acetate and butyl acetate; aprotic polar solvents such as acetonitrile, N,N-dimethylformamide and N-methylpyrrolidone; and chlorinated hydrocarbon solvents such as dichloromethane and chloroform. One of these solvents may be used alone, or two or more may be used in admixture.

When a solvent is used, the amount thereof is not particularly limited, but is preferably from 0.1 to 10.0 liters, and more preferably from 0.5 to 2.0 liters, per mole of Compound (2).

The pressure during the reaction is not particularly limited, although carrying out the reaction at a normal pressure is preferred.

The reaction temperature, although not particularly limited, is preferably between 20°C and 150°C, and more preferably between 50°C and 100°C. The reaction time also is not particularly limited, and is preferably from 1 to 40 hours, and more preferably from 1 to 10 hours.

The reaction is preferably carried out in a nitrogen, argon or other inert gas atmosphere.

A solvent can be used also in the immobilizing step which mixes together a solution of above Compound (1) and an inorganic support, inducing the formation of covalent bonds between silicon atoms on the phenol moiety-containing quaternary phosphonium silane and oxygen atoms on the surface of the inorganic support, and thereby immobilizing the phenol moiety-containing quaternary phosphonium salt on the inorganic support. This solvent is exemplified by the same solvents as those which may be used in the Compound (1) forming step.

In cases where a solvent is used in the immobilizing step, the amount thereof, although not particularly limited, is preferably from 1.0 to 50.0 liters, and more preferably from 5.0 to 20.0 liters, per mole of Compound (2).

The amount of inorganic support used in the immobilizing step is not particularly limited, although given that a higher loading provides a better catalytic activity, the amount per mole of silicon atoms in Compound (1) is preferably from 0.5 to 10.0 kg (which corresponds to from 2.0 mmol/g to 0.1 mmol/g), and more preferably from 0.5 to 5.0 kg (which corresponds to from 2.0 mmol/g to 0.2 mmol/g).

There is no limitation on the pressure in the immobilizing step, although it is preferable for this step to be carried out at a normal pressure.

The reaction temperature, although not particularly limited, is preferably between 20°C and 150°C, and more preferably between 50°C and 120°C. The reaction time also is not particularly limited, but is preferably from 1 to 40 hours, and more preferably from 1 to 10 hours.

The reaction is preferably carried out in a nitrogen, argon or other inert gas atmosphere.

Depending on the target quality, the immobilized phenol moiety-containing quaternary phosphonium salt obtained by the preparation method of the invention can also be used after further purification by filtration, washing, vacuum drying or the like. To obtain a high-purity immobilized phenol moiety-containing quaternary phosphonium salt, purification by filtration, washing or vacuum drying is especially preferred.

Next, a method for synthesizing a cyclic carbonate compound of general formula (7) below (referred to below as "Compound (7)") by carrying out a cycloaddition reaction between an epoxide of general formula (4) below (referred to below as "Compound (4)") and carbon dioxide using the resulting phenolic moiety-containing quaternary phosphonium silane as the catalyst is described.

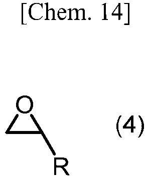

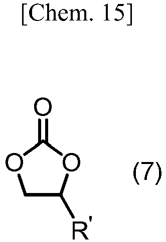

In general formula (4), R is a substituted or unsubstituted monovalent hydrocarbon group of 1 to 18 carbon atoms, preferably 1 to 10 carbon atoms, and more preferably 1 to 5 carbon atoms, which may include a heteroatom, a group of general formula (5) below or a group of general formula (6) below.

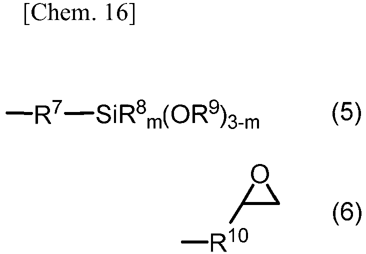

In general formula (4), the monovalent hydrocarbon group represented by R may be linear, branched or cyclic. Specific examples include linear alkyl groups such as methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl and n-octadecyl groups; branched alkyl groups such as isopropyl, isobutyl, sec-butyl, tert-butyl, isopentyl, neopentyl, isohexyl, isoheptyl, isooctyl, tert-octyl, isononyl and isodecyl groups; cyclic alkyl groups such as cyclopentyl and cyclohexyl groups; alkenyl groups such as vinyl, allyl, 1-propenyl, methallyl (2-methyl-2-propenyl) and butenyl groups; aryl groups such as phenyl, tolyl and xylyl groups; and aralkyl groups such as benzyl and phenethyl groups.

Of these, substituted or unsubstituted linear or branched alkyl groups of 1 to 10 carbon atoms, alkenyl groups, aryl groups and aralkyl groups are preferred. Particularly from the standpoint of the availability of the starting materials, alkyl groups of 1 to 6 carbon atoms and aryl groups of 6 to 9 carbon atoms are more preferred; methyl, ethyl, butyl and phenyl groups are even more preferred.

Some or all of the hydrogen atoms on these monovalent hydrocarbon groups may be replaced with other substituents. These substituents are exemplified by the same substituents as mentioned for R¹ and R².

Of these, particularly from the standpoint of the availability of the starting materials, alkoxy groups of 1 to 3 carbon atoms, fluorine atoms, chlorine atoms and phenyl groups are more preferred as the other substituents.

These monovalent hydrocarbon groups may have one, two or more types of heteroatom interposed on the molecular chain, as in the case of ether, carbonyl, amino and sulfide groups.

In general formula (5) above, R⁷ is an unsubstituted divalent hydrocarbon group of 1 to 10 carbon atoms, preferably 1 to 8 carbon atoms, and more preferably 1 to 5 carbon atoms, which may include a heteroatom. The divalent hydrocarbon group of R⁷ is exemplified by the same substituents as R³.

Of these, R⁷ is preferably an alkylene group containing an unsubstituted linear ether group of 3 to 10 carbon atoms. Particularly from the standpoint of the availability of the starting materials, the 3-methyleneoxytrimethylene group and the 8-methyleneoxyoctamethylene group are more preferred.

R⁸ and R⁹ are each independently a substituted or unsubstituted monovalent hydrocarbon group of 1 to 10 carbon atoms, preferably 1 to 5 carbon atoms, and more preferably 1 to 3 carbon atoms. The monovalent hydrocarbon groups represented by R⁸ and R⁹ are exemplified by the same substituents as R¹ and R².

Of these, R⁸ and R⁹ are more preferably unsubstituted linear alkyl groups of 1 to 3 carbon atoms or alkenyl groups. Particularly from the standpoint of the availability of the starting materials, methyl and ethyl groups are even more preferred.

Also, m is an integer from 0 to 3.

In general formula (6), R¹⁰ is a substituted or unsubstituted divalent hydrocarbon group of 1 to 20 carbon atoms, preferably 1 to 10 carbon atoms, and more preferably 1 to 5 carbon atoms, which may include a heteroatom.

The divalent hydrocarbon group represented by R¹⁰ may be linear, branched or cyclic. Specific examples include alkylene groups such as methylene, ethylene, trimethylene, tetramethylene, isobutylene, hexamethylene, octamethylene, decamethylene, dodecamethylene, octadecamethylene, cyclohexylene, neopentylene and methylenecyclohexylene groups; alkenylene groups such as butynylene, propenylene, butenylene, hexenylene and octenylene groups; arylene groups such as the phenylene group; and aralkylene groups such as methylenephenylene and methylenephenylenemethylene groups.

The divalent hydrocarbon groups of R¹⁰ may have one, two or more types of heteroatoms interposed on the molecular chain, as in the case of ether, carbonyl, amino and sulfide groups. Specific examples include oxyalkylene groups such as the methyleneoxydimethyleneoxymethylene and methyleneoxytetramethyleneoxymethylene groups.

Of these, R¹⁰ is preferably an unsubstituted alkylene group of 2 to 10 carbon atoms which may include an ether group. Particularly from the standpoint of the availability of the starting materials, ethylene, tetramethylene, methyleneoxydimethyleneoxymethylene and methyleneoxytetramethyleneoxymethylene groups are more preferred.

Some or all of the hydrogen atoms on these divalent hydrocarbon groups may be replaced with other substituents. These substituents are exemplified by the same substituents as R¹ and R². Of these, particularly from the standpoint of the availability of the starting materials, alkoxy groups of 1 to 3 carbon atoms, phenyl groups and fluorine atoms are more preferred as the other substituents.

Specific examples of Compound (4) include aliphatic epoxides such as ethylene oxide, propylene oxide, 1,2-epoxybutane, 1,2-epoxyhexane, 1,2-epoxyoctane and 1,2-epoxydodecane; aromatic epoxides such as styrene oxide; glycidyl ethers such as allyl glycidyl ether, butyl glycidyl ether and benzyl glycidyl ether; silyl group-containing epoxides such as 3-glycidyloxypropyltrimethoxysilane, 3-glycidyloxypropyldimethoxymethylsilane, 3-glycidyloxypropylmethoxydimethylsilane, 3-glycidyloxypropyltriethoxysilane, 3-glycidyloxypropyldiethoxymethylsilane, 3-glycidyloxypropylethoxydimethylsilane, 8-glycidyloxyoctyltrimethoxysilane, 8-glycidyloxyoctyldimethoxymethylsilane, 8-glycidyloxyoctylmethoxydimethylsilane, 8-glycidyloxyoctyltriethoxysilane, 8-glycidyloxyoctyldiethoxymethylsilane and 8-glycidyloxyoctylethoxydimethylsilane; and difunctional epoxides such as 5-hexadiene diepoxide, 1,7-octadiene diepoxide, ethylene glycol diglycidyl ether, 1,4-butanediol diglycidyl ether, 1,6-hexanediol diglycidyl ether, neopentyl glycol diglycidyl ether, 2,2'-bis(4-glycidyloxyphenyl)propane and resorcinol diglycidyl ether.

A commercially available product may be used as Compound (4), or Compound (4) may be synthesized. In cases where it is synthesized, Compound (4) can be obtained in accordance with a known method, such as that of subjecting an alcohol and epichlorohydrin to a substitution reaction in the presence of a base.

The amount of carbon dioxide used in the cycloaddition reaction is not particularly limited. However, from the standpoint of reducing the volume of exhaust gas, the amount is preferably from 1.0 to 10.0 moles, and more preferably from 1.1 to 3.0 moles, per mole of Compound (4).

The carbon dioxide pressure, although not limited, is preferably from 0.09 to 10.0 MPa. To avoid the need for reaction equipment that can withstand pressurization, the pressure is more preferably from 0.09 to 0.11 MPa.

The amount of phenol moiety-containing quaternary phosphonium silane that can be used in the cycloaddition reaction is not particularly limited, although this is preferably from 0.001 to 0.1 mole, and more preferably from 0.01 to 0.05 mole, per mole of Compound (4).

From the standpoint of lowering energy costs, the cycloaddition reaction is preferably carried out at room temperature (between 1°C and 30°C), and more preferably between 10°C and 30°C. Heating may be carried out in order to shorten the reaction time. The heating temperature in such cases is preferably between 40°C and 100°C, and more preferably between 40°C and 70°C.

The reaction time, although not particularly limited, is preferably from 1 to 40 hours, and more preferably from 1 to 20 hours.

Also, for environmental compatibility, the cycloaddition reaction is preferably carried out in a solvent-free state, although a solvent may be used where necessary. Solvents that may be used include hydrocarbon solvents such as benzene, toluene and xylene; ether solvents such as diethyl ether, tetrahydrofuran, 2-methyltetrahydrofuran, 4-methyltetrahydropyran and dioxane; ester solvents such as ethyl acetate and butyl acetate; aprotic polar solvents such as acetonitrile, N,N-dimethylformamide ad N-methylpyrrolidone; and chlorinated hydrocarbon solvents such as dichloromethane and chloroform. One of these solvents may be used alone, or two or more may be mixed together.

In general formula (7) above, R' is a substituted or unsubstituted monovalent hydrocarbon group of 1 to 18 carbon atoms which may include a heteroatom, a group of general formula (5) below or a group of general formula (8) below. 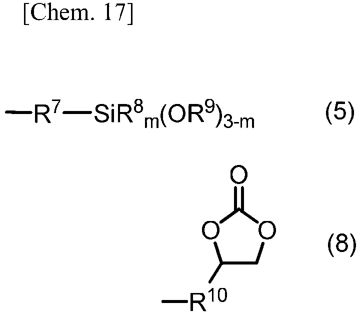 In these formulas, R⁷ to R¹⁰ and m are as defined above.

In above general formula (7), the monovalent hydrocarbon group represented by R' is a substituent similar to cases in which R is a substituted or unsubstituted monovalent hydrocarbon group of 1 to 18 carbon atoms that may include a heteroatom.

Specific examples of Compound (7) include aliphatic cyclic carbonates such as ethylene carbonate, propylene carbonate, 4-ethyl-1,3-dioxolan-2-one, 4-butyl-1,3-dioxolan-2-one, 4-hexyl-1,3-dioxolan-2-one and 4-octyl-1,3-dioxolan-2-one; aromatic cyclic carbonates such as 4-phenyl-1,3-dioxolan-2-one; alkoxymethyl cyclic carbonates such as 4-[(2-propen-1-yloxy)methyl]-1,3-dioxolan-2-one, 4-(butoxymethyl)-1,3-dioxolan-2-one and 4-(benzyloxymethyl)-1,3-dioxolan-2-one; silyl group-containing cyclic carbonates such as 4-[(3-trimethoxysilyl)propoxymethyl]-1,3-dioxolan-2-one, 4-[(3-dimethoxymethylsilyl)propoxymethyl]-1,3-dioxolan-2-one, 4-[(3-methoxydimethylsilyl)propoxymethyl]-1,3-dioxolan-2-one, 4-[(3-triethoxysilyl)propoxymethyl]-1,3-dioxolan-2-one, 4-[(3-diethoxymethylsilyl)propoxymethyl]-1,3-dioxolan-2-one, 4-[(3-ethoxydimethylsilyl)propoxymethyl]-1,3-dioxolan-2-one, 4-[(8-trimethoxysilyl)octoxymethyl]-1,3-dioxolan-2-one, 4-[(8-dimethoxymethylsilyl)octoxymethyl]-1,3-dioxolan-2-one, 4-[(8-methoxydimethylsilyl)octoxymethyl]-1,3-dioxolan-2-one, 4-[(8-triethoxysilyl)octoxymethyl]-1,3-dioxolan-2-one, 4-[(8-diethoxymethylsilyl)octoxymethyl]-1,3-dioxolan-2-one and 4-[(8-ethoxydimethylsilyl)octoxymethyl]-1,3-dioxolan-2-one; and difunctional cyclic carbonates such as 4,4'-(1,2-ethanediyl)bis(1,3-dioxolan-2-one), 4,4'-(1,4-butanediyl)bis(1,3-dioxolan-2-one), 4,4'-[(1,2-ethanediyl)bis(oxymethylene)]bis(1,3-dioxolan-2-one), 4,4'-[(1,4-butanediyl)bis(oxymethylene)]bis(1,3-dioxolan-2-one), 4,4'-[(1,6-hexanediyl)bis(oxymethylene)]bis(1,3-dioxolan-2-one), 4,4'-[(2,2-dimethyl-1,3-propanediyl)bis(oxymethylene)]bis(1,3-dioxolan-2-one), 4,4'-[(1-methylethylidene)bis(4,1-phenyleneoxymethylene)]bis(1,3-dioxolan-2-one) and 4,4'-[(1,3-phenylene)bis(oxymethylene)]bis(1,3-dioxolan-2-one).

The cyclic carbonate compound obtained by the synthesis method of the invention can also be used after purification by various methods of purification, such as distillation, filtration, washing and column separation. To achieve a high purity, purification by distillation is especially preferred.

### EXAMPLES

Examples of the invention and Comparative Examples are given below by way of illustration, although the invention is not limited by these Examples.

The conversion rate of a reaction was taken to be the area ratio percentage between the epoxide starting material and the cyclic carbonate product in gas chromatographic or ¹H-NMR analysis.

The cyclic carbonate purity is a value measured under the following gas chromatography measurement conditions.

### [Gas Chromatography Measurement Conditions]

| | |
|---|---|
| Gas chromatograph: | GC-2014 (from Shimadzu Corporation) |
| Packed column: | Silicone SE-30 (from GL Sciences Inc.) |
| Detector: | TCD |
| Detector temperature: | 300°C |
| Injection port temperature: | 300°C |
| Temperature program: | 70°C (0 min) → 10°C/min → 300°C (10 min) |
| Carrier gas: | helium (50 mL/min) |
| Amount of sample injected: | 1 µL |

### [1] Preparation of Immobilized Phenol Moiety-Containing Quaternary Phosphonium Salts

### [Example 1]

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 37.0 g (100.0 mmol) of 4-hydroxy-2-(triphenylphosphonium) phenolate and 100 mL of N,N-dimethylformamide, and the flask contents were stirred at 80°C. While regulating the temperature to an internal temperature of between 75°C and 85°C, 24.3 g (100 mmol) of (3-bromopropyl)trimethoxysilane was added dropwise, after which the flask contents were stirred at 80°C for 2 hours, giving a reaction mixture containing (2-[3-(trimethoxysilyl)propoxy]-5-hydroxyphenyl)triphenylphophonium bromide as the main ingredient.

The resulting reaction mixture was diluted with 220 mL of toluene, following which it was added to 200.0 g of silica gel that had been dried for 2 hours at 120°C and 30 Pa (trade name: Wakogel^{®} C-200, from Fujifilm Wako Pure Chemical Corporation) and stirred at 80°C for 3 hours. The reaction mixture was cooled to room temperature, following which filtration was carried out by passing the reaction mixture through a PTFE membrane (pressure, 0.3 MPa) having a pore size of 0.2 µm. The recovered silica gel was washed three times with a mixture of 140 mL of acetone and 60 mL of methanol, giving a treated silica gel. The treated silica gel was dried at 80°C and 30 Pa for 2 hours, yielding 236.0 g of an immobilized phenol moiety-containing quaternary phosphonium salt.

The resulting immobilized phenol moiety-containing quaternary phosphonium salt was confirmed by elemental analysis to have a carbon content of 8.26 wt%. The content of phosphorus included in the immobilized phenol moiety-containing quaternary phosphonium salt, as measured by fluorescence x-ray spectrometry (XRF), was 0.65 wt%. The content of bromine included in the immobilized phenol moiety-containing quaternary phosphonium salt, as measured by potentiometric titration, was 1.82 wt%. These analytic results demonstrate that the loading of phosphorus atoms in the immobilized phenol moiety-containing quaternary phosphonium salt was 0.21 mmol/g and the loading of bromine atoms was 0.23 mmol/g.

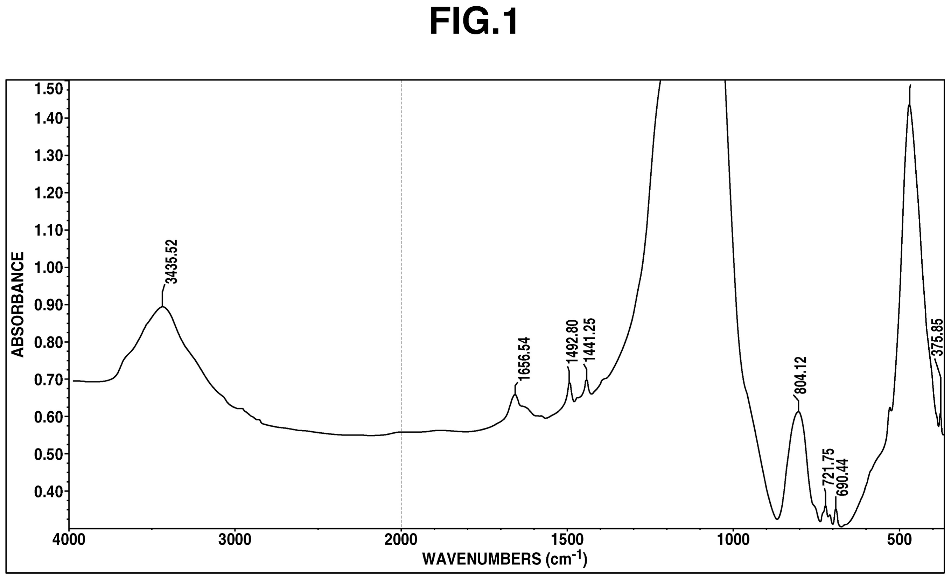

The IR spectrum of the resulting immobilized phenol moiety-containing quaternary phosphonium salt was measured. Those results are shown in FIG. 1.

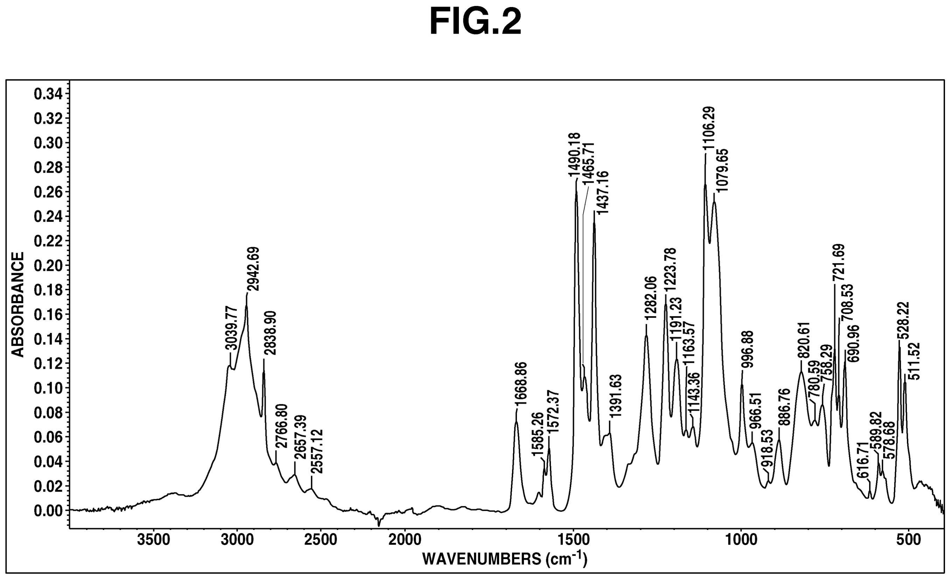

The IR spectrum of the (2-[3-(trimethoxysilyl)propoxy]-5-hydroxyphenyl)-triphenylphosphonium bromide synthesized by the same method as in Example 1 was measured. The results are shown in FIG. 2.

As shown in FIGS. 1 and 2, characteristic peaks are observed at 1441, 1492 and 1656 cm⁻¹ in the immobilized phenol moiety-containing quaternary phosphonium salt, and peaks corresponding to these are observed at, respectively, 1437, 1490 and 1668 cm⁻¹ in (2-[3-(trimethoxysilyl)propoxy]-5-hydroxyphenyl)triphenylphosphonium bromide. These results indicate that quaternary phosphonium moieties are immobilized on the silica gel.

### [2] Synthesis of Cyclic Carbonate Compounds

### [Example 2-1]

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 37.0 g (P: 7.8 mmol) of the immobilized phenol moiety-containing quaternary phosphonium salt synthesized in Example 1 and 57.1 g (500 mmol) of allyl glycidyl ether, and the flask contents were stirred. Next, while regulating the temperature to an internal temperature of 27°C, the contents were stirred for 20 hours while bubbling carbon dioxide through the reaction solution at normal pressure and a rate of 28 mL/min (1,500 mmol over 20 hours).

A small amount of the reaction mixture was sampled and the rate of conversion was determined by gas chromatography. The rate of conversion from allyl glycidyl ether to 4-[(2-propen-1-yloxy)methyl]-1,3-dioxolan-2-one was found to be 70.1%. Filtration was carried out by passing the reaction mixture through a PTFE membrane (pressure, 0.3 MPa) having a pore size of 0.2 µm, following which the recovered silica gel was washed three times with 37 mL of toluene. The resulting filtrate was distilled at 0.2 kPa, giving 4-[(2-propen-1-yloxy)methyl]-1,3-dioxolan-2-one at an isolated yield of 64.0% and a purity of at least 99.9%.

### [Example 2-2]

A four-neck flask equipped with a stirrer, a reflux condenser, a dropping funnel and a thermometer was purged with nitrogen and, while passing nitrogen gas over the open end at the top of the reflux condenser so as to prevent outside air from entering, was charged with 47.6 g (P: 10.0 mmol) of the immobilized phenol moiety-containing quaternary phosphonium salt synthesized in Example 1 and 29.0 g (500 mmol) of propylene oxide and 50 mL of toluene, and the flask contents were stirred. Next, while regulating the temperature to an internal temperature of 25°C, the contents were stirred for 20 hours while bubbling carbon dioxide through the reaction solution at normal pressure and a rate of 28 mL/min (1,500 mmol over 20 hours).

A small amount of the reaction mixture was sampled and the rate of conversion was determined by gas chromatography. The rate of conversion from propylene oxide to propylene carbonate was found to be 99.6%. Filtration was carried out by passing the reaction mixture through a PTFE membrane (pressure, 0.3 MPa) having a pore size of 0.2 µm, following which the recovered silica gel was washed three times with 50 mL of toluene. The resulting filtrate was distilled at 1.2 kPa, giving propylene carbonate at an isolated yield of 79.4% and a purity of 99.4%.

### [Example 2-3]

A four-neck flask equipped with a stirrer, a reflux condenser, a dropping funnel and a thermometer was purged with nitrogen and, while passing nitrogen gas over the open end at the top of the reflux condenser so as to prevent outside the entry of air, was charged with 25.0 g (P: 5.3 mmol) of the immobilized phenol moiety-containing quaternary phosphonium salt synthesized in Example 1 and 50.0 g (384 mmol) of butyl glycidyl ether, and the flask contents were stirred. Next, while regulating the temperature to an internal temperature of 70°C, the contents were stirred for 8 hours while bubbling carbon dioxide through the reaction solution at normal pressure and a rate of 53.8 mL/min (1,152 mmol over 8 hours).

A small amount of the reaction mixture was sampled and the rate of conversion was determined by gas chromatography. The rate of conversion from butyl glycidyl ether to 4-butoxymethyl-1,3-dioxolan-2-one was found to be 93.2%. Filtration was carried out by passing the reaction mixture through a PTFE membrane (pressure, 0.3 MPa) having a pore size of 0.2 µm, following which the recovered silica gel was washed three times with 50 mL of toluene. The resulting filtrate was distilled at 0.2 kPa, giving 4-butoxymethyl-1,3-dioxolan-2-one at an isolated yield of 87.5% and a purity of at least 99.9%.

In addition, the reusability as a silica gel catalyst of the immobilized phenol moiety-containing quaternary phosphonium salt recovered as described above was confirmed.

Specifically, the recovered silica gel was washed three times with toluene as described above, following which it was dried for 2 hours at 80°C and 30 Pa. Using this silica gel, the cycloaddition of butyl glycidyl ether was carried out by repeating the same procedure as described above four times. As a result, the rates of conversion from butyl glycidyl ether to 4-butoxymethyl-1,3-dioxolan-2-one during the second to fifth uses of the catalyst were respectively 84.7%, 79.4%, 77.0% and 73.8%.

### [Example 2-4]

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 62.5 g (P: 13.1 mmol) of the immobilized phenol moiety-containing quaternary phosphonium salt synthesized in Example 1 and 118.0 g (500 mmol) of 3-glycidyloxypropyltrimethoxysilane, and the flask contents were stirred. Next, while regulating the temperature to an internal temperature of 70°C, the contents were stirred for 8 hours while bubbling carbon dioxide through the reaction solution at normal pressure and a rate of 62.2 mL/min (1,334 mmol over 8 hours).

A small amount of the reaction mixture was sampled and the rate of conversion was determined by gas chromatography. The rate of conversion from 3-glycidyloxypropyltrimethoxysilane to 4-[(3-trimethoxysilyl)propoxymethyl]-1,3-dioxolan-2-one was found to be 88.2%. The reaction mixture was cooled to room temperature and filtration was carried out by passing the reaction mixture through a PTFE membrane (pressure, 0.3 MPa) having a pore size of 0.2 µm, following which the recovered silica gel was washed three times with 50 mL of toluene. The resulting filtrate was distilled at 30 Pa, yielding 4-[(3-trimethoxysilyl)propoxymethyl]-1,3-dioxolan-2-one at an isolated yield of 77.6% and a purity of 99.3%.

### [Example 2-5]

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 42.0 g (P: 8.8 mmol) of the immobilized phenol moiety-containing quaternary phosphonium salt synthesized in Example 1 and 92.0 g (500 mmol) of 1,2-epoxydodecane, and the flask contents were stirred. Next, while regulating the temperature to an internal temperature of 70°C, the contents were stirred for 8 hours while bubbling carbon dioxide through the reaction solution at normal pressure and a rate of 62.2 mL/min (1,334 mmol over 8 hours).

A small amount of the reaction mixture was sampled and the rate of conversion was determined by gas chromatography. The rate of conversion from 1,2-epoxydodecane to 4-decyl-1,3-dioxolan-2-one was found to be 97.4%. The reaction mixture was cooled to room temperature and filtration was carried out by passing the reaction mixture through a PTFE membrane (pressure, 0.3 MPa) having a pore size of 0.2 µm, following which the recovered silica gel was washed three times with 50 mL of toluene. The resulting filtrate was distilled at 30 Pa, yielding 4-decyl-1,3-dioxolan-2-one at an isolated yield of 91.4% and a purity of 99.3%.

### [Example 2-6]

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 62.5 g (P: 13.1 mmol) of the immobilized phenol moiety-containing quaternary phosphonium salt synthesized in Example 1 and 108.1 g (500 mmol) of neopentyl glycol diglycidyl ether, and the flask contents were stirred. Next, while regulating the temperature to an internal temperature of 70°C, the contents were stirred for 16 hours while bubbling carbon dioxide through the reaction solution at normal pressure and a rate of 62.2 mL/min (2,668 mmol over 16 hours).

The reaction mixture, while being held at a temperature of 70°C, was passed through a PTFE membrane (pressure, 0.3 MPa) having a pore size of 0.2 µm, following which the recovered silica gel was washed three times with 50 mL of acetone. The resulting filtrate was concentrated with an evaporator, following which it was dried for 1 hour at 100°C and 30 Pa, giving a product composed primarily of 4,4'-[(2,2-dimethyl-1,3-propanediyl)bis(oxymethylene)]bis(1,3-dioxolan-2-one) at a yield of 94.6%.

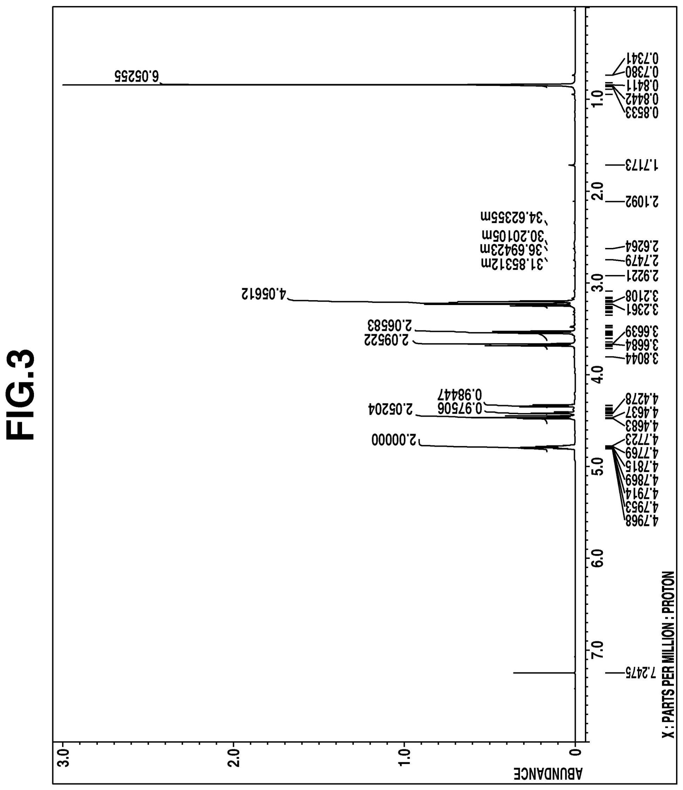

The ¹H-NMR spectrum (heavy chloroform solvent) of the resulting 4,4'-[(2,2-dimethyl-1,3-propanediyl)bis(oxymethylene)]bis(1,3-dioxolan-2-one) was measured. The results are shown in FIG. 3.

As shown in FIG. 3, peaks for the epoxide moiety are very slightly observable in the ¹H-NMR spectrum. From the area ratio with peaks for the cyclic carbonate moiety, the rate of conversion from the starting epoxide to the corresponding cyclic carbonate was found to be 98.3%. Moreover, the product included 1.5% of unidentified substances as impurities. The purity of the resulting 4,4'-[(2,2-dimethyl-1,3-propanediyl)bis(oxymethylene)]bis(1,3-dioxolan-2-one) was 96.9%.

### [Comparative Example 1]

A carbonate conversion reaction was carried out, under the same reaction conditions as in Example 2-1, on allyl glycidyl ether using the silica gel-immobilized phosphonium bromide salt described in Patent Document 1. Aside from changing the type of silica gel to Wakogel^{®} C-200 from Fujifilm Wako Pure Chemical Corporation, a silica gel-immobilized phosphonium bromide salt derived from tri(p-tolyl)phosphine and 3-bromopropyltriethoxysilane was synthesized by the method described in Embodiment 2c of Patent Document 1.

A four-neck flask equipped with a stirrer, a reflux condenser, a dropping funnel and a thermometer was purged with nitrogen and, while passing nitrogen gas over the open end at the top of the reflux condenser so as to prevent the entry of outside air, was charged with 37.0 g of the silica gel-immobilized phosphonium bromide salt obtained as described above and 57.1 g (500 mmol) of allyl glycidyl ether, and the flask contents were stirred. Next, while regulating the temperature to an internal temperature of 27°C, the contents were stirred for 20 hours while bubbling carbon dioxide through the reaction solution at normal pressure and a rate of 28 mL/min (1,500 mmol over 20 hours).

A small amount of the reaction mixture was sampled and the rate of conversion was determined by gas chromatography. The rate of conversion from allyl glycidyl ether to 4-[(2-propen-1-yloxy)methyl]-1,3-dioxolan-2-one was found to be 34.9%. The reaction mixture was filtered by passing it through a PTFE membrane (pressure, 0.3 MPa) having a pore size of 0.2 µm, following which the recovered silica gel was washed three times with 37 mL of toluene. The resulting filtrate was distilled at 0.2 kPa, giving 4-[(2-propen-1-yloxy)methyl]-1,3-dioxolan-2-one at an isolated yield of 20.5% and a purity of 99.8%.

As mentioned above, in both Examples 2-1 and 2-2, although the reaction was carried out under mild conditions of normal pressure and room temperature, the corresponding cyclic carbonate compound formed at a high rate of conversion and moreover was isolated in a high yield and at high purity. These results demonstrate that the immobilized phenol moiety-containing quaternary phosphonium salt of the invention exhibits an excellent catalytic activity even under mild conditions.

In Examples 2-3 to 2-6, when the reaction temperature was set to 70°C in order to shorten the aging time, the above cycloaddition reaction proceeded more smoothly and, in each of these cases, a cyclic carbonate compound was obtained in a high yield and at high purity.

In Example 2-3, the reusability of an immobilized phenol moiety-containing quaternary phosphonium salt according to the invention was confirmed. When catalyst reuse was repeatedly carried out in a cycloaddition reaction between butyl glycidyl ether and carbon dioxide, although the rate of conversion to 4-butoxymethyl-1,3-dioxolan-2-one gradually decreased, it was possible to recover and reuse the immobilized phenol moiety-containing quaternary phosphonium salt without a major accompanying loss in the catalytic activity.

By contrast, in Comparative Example 1, using the silica gel-immobilized phosphonium bromide salt described in Patent Document 1 as the catalyst, the same cycloaddition reaction between allyl glycidyl ether and carbon dioxide as in Example 2-1 was carried out at normal pressure and room temperature, but the 4-[(2-propen-1-yloxy)methyl]-1,3-dioxolan-2-one product was formed only at a low rate of conversion, and the isolated yield was very low.

## Claims

1. A phenol moiety-containing quaternary phosphonium salt immobilized on an inorganic support, which immobilized quaternary phosphonium salt comprises at least a phenol moiety-containing quaternary phosphonium silane of general formula (1) below 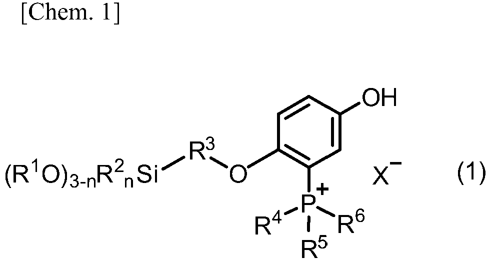 (wherein R¹ and R² are each independently a substituted or unsubstituted monovalent hydrocarbon group of 1 to 10 carbon atoms, R³ is an unsubstituted divalent hydrocarbon group of 1 to 10 carbon atoms that may include a heteroatom, R⁴ to R⁶ are each independently a substituted or unsubstituted monovalent hydrocarbon group of 1 to 10 carbon atoms, n is an integer from 0 to 2, and X is a halogen atom) and an inorganic support, wherein the quaternary phosphonium salt is immobilized on the inorganic support via covalent bonds between silicon atoms on the quaternary phosphonium silane and oxygen atoms present on a surface of the inorganic support.

2. A method for preparing an immobilized, phenol moiety-containing quaternary phosphonium salt of claim 1, comprising the steps of, in order:
reacting a haloalkyl alkoxysilane compound of general formula (2) below 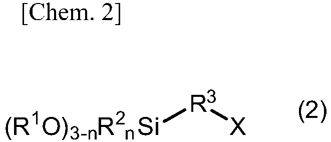
(wherein R¹ to R³, X and n are as defined above)
with a phosphabetaine compound of general formula (3) below 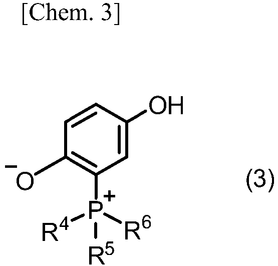
(wherein R⁴ to R⁶ are as defined above)
to form a phenol moiety-containing quaternary phosphonium silane of general formula (1) below 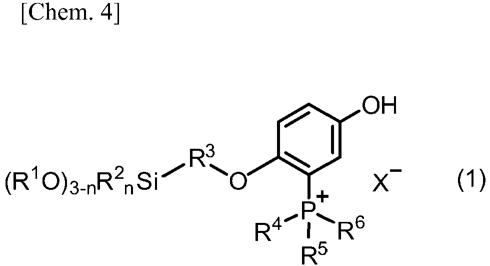
(wherein R¹ to R⁶, X and n are as defined above); and
mixing the resulting phenol moiety-containing quaternary phosphonium silane with an inorganic support to form covalent bonds between silicon atoms on the phenolic moiety-containing quaternary phosphonium silane and oxygen atoms present on a surface of the inorganic support, thereby immobilizing the quaternary phosphonium silane on the inorganic support.

3. A method for synthesizing a cyclic carbonate compound of general formula (7) below 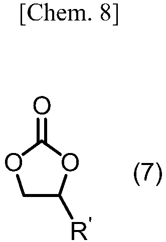
[wherein R' is a substituted or unsubstituted monovalent hydrocarbon group of 1 to 18 carbon atoms which may include a heteroatom, a group of general formula (5) below
[Chem. 9]
-R⁷-SiR⁸ₘ(OR⁹)₃₋ₘ (5)
(wherein R⁷ is an unsubstituted divalent hydrocarbon group of 1 to 10 carbon atoms which may include a heteroatom, R⁸ and R⁹ are each independently a substituted or unsubstituted monovalent hydrocarbon group of 1 to 10 carbon atoms, and m is an integer from 0 to 3) or a group of general formula (8) below 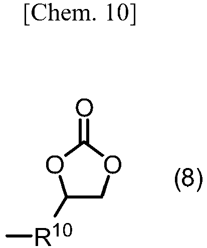
(wherein R¹⁰ is a substituted or unsubstituted divalent hydrocarbon group of 1 to 20 carbon atoms which may include a heteroatom)], which method comprises the step of carrying out, in the presence of a catalyst, a cycloaddition reaction between an epoxide of general formula (4) below 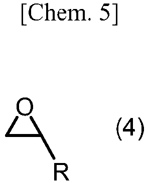
[wherein R is a substituted or unsubstituted monovalent hydrocarbon group of 1 to 18 carbon atoms which may include a heteroatom, a group of general formula (5) below
[Chem. 6]
-R⁷-SiR⁸ₘ(OR⁹)₃₋ₘ (5)
(wherein R⁷ to R⁹ and m are as defined above) or a group of general formula (6) below 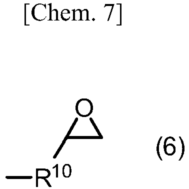
(wherein R¹⁰ is as defined above)] and carbon dioxide,
wherein the catalyst used to synthesize the cyclic carbonate compound is the immobilized, phenol moiety-containing quaternary phosphonium salt of claim 1.
